Europäisches Patentamt

European Patent Office

Office européen des brevets

(11) Publication number: **0 081 896
B1**

(12) **EUROPEAN PATENT SPECIFICATION**

(45) Date of publication of patent specification: **15.04.87**

(21) Application number: **82304017.5**

(22) Date of filing: **29.07.82**

(51) Int. Cl.⁴: **A 61 K 9/06, A 61 K 47/00**

(54) **Waterless thixotropic composition.**

(30) Priority: **14.12.81 US 330541**

(43) Date of publication of application:
**22.06.83 Bulletin 83/25**

(45) Publication of the grant of the patent:
**15.04.87 Bulletin 87/16**

(84) Designated Contracting States:
**AT BE CH DE FR GB IT LI LU NL SE**

(56) References cited:
**CA-A- 952 430**

**CHEMICAL ABSTRACTS, vol. 75, no. 5, August
2, 1971, page 358, no. 34089z, Columbus, Ohio,
US C.A. ELLIGER et al.: "Starch gel modifiers
from 12-hydroxystearic acid"**

(73) Proprietor: **MERCK & CO. INC.
126, East Lincoln Avenue P.O. Box 2000
Rahway New Jersey 07065 (US)**

(72) Inventor: **Zingerman, Joel R.
221 Avon Road
Wastfield New Jersey 07090 (US)**

(74) Representative: **Crampton, Keith John Allen
et al
D YOUNG & CO 10 Staple Inn
London WC1V 7RD (GB)**

Courier Press, Leamington Spa, England.

## Description

This invention relates to medicament formulations and compositions that are administered to animals. More particularly it relates to thixotropic compositions of medicament that are usually administered by means of an incremental delivery device. Even more particularly, but not exclusively, the invention relates to stable semi-solid formulations of macrolide antibiotics such as C-076 which is described in U.S. Patent Specification US—A—4 199 579.

Chemical Abstracts, Volume 75 (1971), 34089 Z, discloses that surfactants derived from 12-hydroxystearic acid impart a high viscosity to a starch gel, and that this could be advantageous in suspending fruit or nuts in pudding and pie fillings.

Canadian Patent CA—A—952 430 discloses anhydrous gel formulations suitable as suspending or thickening agents and containing a water-soluble polymer, e.g. a cellulose derivative, and a glycol, e.g. propylene glycol, optionally together with surfactants such as fatty acid monoglycerides.

In the delivery of medicaments to animals it has been found particularly difficult to administer doses in the form of tablets or boluses. The animals tend to reject the dose unless it is thrust so deeply into their throats that they cannot block ingestion with their tongues or otherwise. In practice this is difficult for one other than a trained animal handler to accomplish. Other available means of administration include dosing the drinking water or the feed. In administration through drinking water, stability problems of the drug are sometimes encountered, and administration through either feed or water can result in unacceptable variations of dosage because individual animals's food and water requirements are not uniform.

One desirable means for administering medicament to an animal is to squeeze an incremental dose of semi-solid medicament in the form of a paste or gel into the animal's mouth, most suitably onto the tongue. A paste that is too sticky for the animal to expel is ultimately ingested. A desirable characteristic of a satisfactory paste composition is that it be thixotropic; that is, the composition is fluid only when pressure or shear is applied during the dosing procedure. This allows for easy fluid-like administration without the paste's leaking from the undisturbed delivery cartridge. Another desirable characteristic is that the formulation should be sticky enough to avoid any loss of the formulation once administered.

The paste, of course, must also be palatable to the animal to ensure that it is totally consumed after administration. If the taste is severely objectionable, the animal attempts to work the dose from the mouth, sometimes with partial success even though it tends to adhere to the tongue.

A paste should be free of entrained air so as to eliminate sponginess, which leads to two problems. First, there can be incorrect dose delivery, since the compression of the formulation takes place at the instant when pressure is applied. Also, slow expansion back to the normal volume can result in after-dose oozing from the delivery device. Another desirable characteristic is that the paste should incorporate a fairly high concentration of drug so as to maximize the number of dosages deliverable from a cartridge.

While many dosage formulations for many drugs are available, most of them contain a sufficient amount of water for use as a solvent or as an ingredient required for gel formation. These formulations are impracticable for use with any medicament or other component that is sensitive to chemical attack by the water, e.g. hydrolysis or any other means whereby decomposition occurs, thus altering the therapeutic index of the drug.

The delivery techniques of pastes are well known and need not be dealt with here in detail. In general, in horses the paste is deposited on the posterior portion of the tongue through the interdental space on either side of the head. In cattle the dose can be thrust directly onto the tongue.

The present invention provides an essentially anhydrous gel formulation that can be administered to animals comprising from 0.5 to 10% by weight of a drug having a therapeutic or prophylactic effect on a condition affecting an animal, from 2 to 25% by weight of a hydroxylated fatty acid ester of glycerin, from 55 to 97.2% by weight of a glycol or glycerin, and from 0.3 to 15% by weight of a water-soluble polymer.

The preferred paste formulation of this invention comprises a triester or glycerin and a hydroxylated fatty acid in which the fatty acid residue is from 10 to 22 carbon atoms in length and has at least one hydroxy substituent. The especially preferred ester is trihydroxostearin which is a triester of glycerin and hydroxystearic acid. The second component of the system is an alkylene dihydric alcohol such as a $C_{3-6}$ alkylene glycol, a $C_{4-6}$ alkylene pinacol, glycerol, or a polyalkylene glycol having a molecular weight in the range 200 to 2000. The preferred glycols are propylene glycol and polyethylene glycol. The term "alkylene" as used herein means an alkylene having fewer than six carbon atoms. The third component is a water-soluble polymer, most suitably a cellulose derivative. The fourth component is the medicament.

The molecular weight of the water-soluble polymer is not especially material to the invention. However, low molecular weights appear to be desirable since they impart less stringiness to the paste formulation. To this end, molecular weights of less than 100,000, especially 50,000 to 60,000, are preferred.

Generally all polymers that are soluble in water as well as in organic solvents such as lower alcohols and are generally recognized as safe are appropriate for use in this composition. Such polymers include $C_{1-4}$ alkyl ethers and esters of cellulose derivatives, such as hydroxypropyl cellulose and hydroxypropylmethyl cellulose;

carboxyvinyl polymers available under the trade name Carbopol® from B. F. Goodrich; polyvinyl pyrrolidone; and poly(methylvinylether maleic anhydride), which is available under the trade name Gantrez. The preferred polymer is hydroxypropyl cellulose.

Colours, flavours or opaquing agents such as titanium dioxide can also be added. As the medicament portion of the composition increases, it is more desirable to reduce the glycol content rather than the fat or polymer components. Thus, in a heavily drug loaded paste, there would be, by weight:

| | |
|---|---|
| hydroxylpropyl cellulose | 5.0% |
| trihydroxystearin | 12.0% |
| medicament | 10.0% |
| propylene glycol | 73.0% |

The more suitable compositions contain, by weight, 10 to 15% of hydroxylated fatty acid ester, 3 to 8% of water-soluble polymer, 0.1 to 5% of medicament, the remainder being glycol.

In a preferred composition there is, by weight:

| | |
|---|---|
| c-076 | 0.5% |
| hydroxypropyl cellulose (Klucel EF) | 5.0% |
| trihydroxystearin | 12.0% |
| propylene glycol | 82.5% |

In this preferred composition sufficient titanium dioxide can also be added to render the composition opaque and thereby result not only in a more elegant paste, but one that transmits ultra-violet light to a lesser degree and protects the medicament from UV irradiation. To the total weight of the composition is added from 0.2 to 5% titanium dioxide.

To prepare the formulation, the drug and polymers are dissolved, or, if complete solubility is not attained, are mixed homogeneously until the mixture is combined with the glycol. To achieve dissolution or homogeniety, it is preferable that the drug and polymer be of a small particle size or powdered. If dissolution upon trial is found to be incomplete, then to obtain as complete a dispersal as practical, the polymer or drug is suitably micronized prior to admixing with glycol. Drugs or polymers that are liquids of course pose fewer admixing problems. After dispersal of the drug and polymer in the glycol is complete, the hydroxylated fatty acid ester is added.

While agitating vigorously, suitably with the aid of a high-speed impeller, the hydroxylated fatty acid ester is added in increments so that complete dispersal of the newly added increment is obtained before addition of more hydroxylated fatty acid ester. This addition of the hydroxylated fatty acid ester is desirably accompanied by heating the admixture to 40—65°C, preferably 55—60°C. If heating is not required to achieve dissolution, it is nevertheless generally preferable to heat the admixture of polymer, glycol hydroxylated fatty acid ester and drug to 50—65°C, preferably 55—60°C, to improve the viscosity of the formulation and achieve optimum dispersal. It is at this stage that any flavouring, colouring or opaquing agents can be added and dispersed throughout the formulation. After cooling, the formulation is packaged in suitable containers.

**Claims for Contracting States: BE—SE**

1. An essentially anhydrous gel formulation that can be administered to animals comprising from 0.5 to 10% by weight of a drug having a therapeutic or prophylactic effect on a condition affecting an animal, from 2 to 25% by weight of a hydroxylated fatty acid ester of glycerin, from 55 to 97.2% by weight of a glycol or glycerin, and from 0.3 to 15% by weight of a water-soluble polymer.

2. A formulation according to claim 1 in which the glycol is polyethylene glycol.

3. A formulation according to claim 1 in which the glycol is propylene glycol.

4. A formulation according to any one of claims 1 to 3 in which the hydroxylated fatty acid ester has from 10 to 22 carbon atoms in the fatty acid residue.

5. A formulation according to claim 4 in which the ester has from 10 to 18 carbon atoms and at least one hydroxy substituent in the fatty acid residue.

6. A formulation according to claim 5 in which the hydroxylated fatty acid is trihydroxystearin.

7. A formulation according to any one of claims 1 to 6 in which the drug is the macrolide C-076.

8. A formulation according to any one of claims 1 to 7 in which the polymer is a $C_{1-4}$ alkyl ether or ester of cellulose.

9. A formulation according to any one of claims 1 to 8 in which the polymer has a molecular weight of less than 100,000.

10. A method of preparing a gel for carrying animal medications comprising admixing water-soluble polymer with a drug for treating a condition affecting an animal and a glycol or glycerin, optionally heating the admixture and adding in increments with agitation a fatty acid ester of glycerin.

**Claims for Contracting State: AT**

1. A method of preparing an essentially anhydrous gel formulation that can be administered to animals comprising mixing homogeneously from 0.5 to 10% by weight of a drug having a therapeutic or prophylactic effect on a condition affecting an animal and from 0.3 to 15% by weight of a water-soluble polymer with from 55 to 97.2% by weight of a glycol or glycerin, and then adding gradually from 2 to 25% by weight of a hydroxylated fatty acid ester of glycerin.

2. A method according to claim 1 in which the glycol is polyethylene glycol.

3. A method according to claim 1 in which the glycol is propylene glycol.

4. A method according to any one of claims 1 to 3 in which the hydroxylated fatty acid ester has from 10 to 22 carbon atoms in the fatty acid residue.

5. A method according to claim 4 in which the ester has from 10 to 18 carbon atoms and at least one hydroxy substituent in the fatty acid residue.

6. A method according to claim 5 in which the hydroxylated fatty acid is trihydroxystearin.

7. A method according to any one of claims 1 to 6 in which the drug is the macrolide C-076.

8. A method according to any one of claims 1 to 7 in which the polymer is a $C_{1-4}$ alkyl ether or ester of cellulose.

9. A method according to any one of claims 1 to 8 in which the polymer has a molecular weight of less than 100,000.

10. A method of preparing a gel for carrying animal medications comprising admixing water-soluble polymer with a drug for treating a condition affecting an animal and a glycol or glycerin, optionally heating the admixture and adding in increments with agitation a fatty acid ester of glycerin.

**Patentansprüche für die Vertragsstaaten: BE—SE**

1. Eine im wesentlichen wasserfreie Gelformulierung, die an Tiere verabreicht werden kann, enthaltend 0,5 bis 10 Gew.-% eines Arzneimittels, das eine therapeutische oder prophylaktische Wirkung auf einen, ein Tier beeinflussenden Zustand ausübt, 2 bis 25 Gew.-% eines hydroxylierten Fettsäureesters von Glycerin, 55 bis 97,2 Gew.-% eines Glykols oder Glycerins, und 0,3 bis 15 Gew.-% eines wasserlöslichen Polymers.

2. Eine Formulierung nach Anspruch 1, in welcher das Glykol Polyethylenglykol ist.

3. Eine Formulierung nach Anspruch 1, in welcher das Glykol Propylenglykol ist.

4. Eine Formulierung nach einem der Ansprüche 1 bis 3, in welcher der hydroxylierte Fettsäureester 10 bis 22 Kohlenstoffatome in dem Fettsäurerest enthält.

5. Eine Formulierung nach Anspruch 4, in welcher der Ester 10 bis 18 Kohlenstoffatome und wenigstens einen Hydroxysubstituenten in dem Fettsäurerest aufweist.

6. Eine Formulierung nach Anspruch 5, in welcher die hydroxylierte Fettsäure Trihydroxystearin ist.

7. Eine Formulierung nach einem der Ansprüche 1 bis 6, in welcher das Arzneimittel das Makrolid C-076 ist.

8. Eine Formulierung nach einem der Ansprüche 1 bis 7, in welcher das Polymer ein $C_{1-4}$-Alkylether oder -Ester von Cellulose ist.

9. Eine Formulierung nach einem der Ansprüche 1 bis 8, in welcher das Polymer ein Molekulargewicht von weniger als 100.000 hat.

10. Ein Verfahren zur Herstellung eines Gels als Träger von Tiermedikationen, umfassend das Vermischen von wasserlöslichem Polymer mit einem Arzneimittel zur Behandlung eines ein Tier beeinflussenden Zustandes, und einem Glykol oder Glycerin, gegebenenfalls das Erhitzen des Gemisches und das Zugeben von Teilmengen eines Fettsäureesters von Glycerin unter Rühren.

**Patentansprüche für die Vertragsstaaten: AT**

1. Verfahren zur Herstellung einer im wesentlichen wasserfreien Gelformulierung, die an Tiere verabreicht werden kann, umfassend das homogene Vermischen von 0,5 bis 10 Gew.-% eines Arzneimittels, das eine therapeutische oder prophylaktische Wirkung auf einen, ein Tier beeinflussenden Zustand ausübt, und von 0,3 bis 15 Gew.-% eines wasserlöslichen Polymers mit 55 bis 97,2 Gew.-% eines Glykols oder Glycerins, und dann das allmähliche Zugeben von 2 bis 25 Gew.-% eines hydroxylierten Fettsäureesters von Glycerin.

2. Ein Verfahren nach Anspruch 1, bei welchem das Glykol Polyethylenglykol ist.

3. Ein Verfahren nach Anspruch 1, bei welchem das Glykol Propylenglykol ist.

4. Ein Verfahren nach einem der Ansprüche 1 bis 3, bei welchem der hydroxylierte Fettsäureester 10 bis 22 Kohlenstoffatome in dem Fettsäurerest enthält.

5. Ein Verfahren nach Anspruch 4, bei welchem der Ester 10 bis 18 Kohlenstoffatome und wenigstens einen Hydroxysubstituenten in dem Fettsäurerest aufweist.

6. Ein Verfahren nach Anspruch 5, bei welchem die hydroxylierte Fettsäure Trihydroxystearin ist.

7. Ein Verfahren nach einem der Ansprüche 1 bis 6, bei welchem das Arzneimittel das Makrolid C-076 ist.

8. Ein Verfahren nach einem der Ansprüche 1 bis 7, bei welchem das Polymer ein $C_{1-4}$-Alkylether oder Ester von Cellulose ist.

9. Ein Verfahren nach einem der Ansprüche 1 bis 8, bei welchem das Polymer ein Molekulargewicht von weniger als 100.000 hat.

10. Ein Verfahren zur Herstellung eines Gels als Träger von Tiermedikationen, umfassend das Vermischen von wasserlöslichem Polymer mit einem Arzneimittel zur Behandlung eines ein Tier beeinflussenden Zustandes, und einem Glykol oder Glycerin, gegebenenfalls das Erhitzen des Gemisches und das Zugeben von Teilmengen eines Fettsäureesters von Glycerin unter Rühren.

**Revendications pour les Etats Contractants: BE—SE**

1. Formulation de gel essentiellement anhydre qui peut être administrée aux animaux, comprenant 0,5 à 10% en poids d'un médicament ayant un effet thérapeutique ou prophylactique sur un état affectant un animal, de 2 à 25% en poids d'un ester d'acide gras hydroxylé et de glycérine, de 55 à 97,2% en poids d'un glycol ou de glycérine et de 0,3 à 15% en poids d'un polymère soluble dans l'eau.

2. Formulation selon la revendication 1, caractérisée en ce que le glycol est le polyéthylèneglycol.

3. Formulation selon la revendication 1, caractérisée en ce que le glycol est le propylèneglycol.

4. Formulation selon l'une quelconque des revendications 1 à 3, caractérisée en ce que l'ester d'acide gras hydroxylé possède de 10 à 22 atomes de carbone dans le résidu acide gras.

5. Formulation selon la revendication 4, caractérisée en ce que l'ester possède de 10 à 18 atomes de carbone et au moins un substituant hydroxyle dans le résidu acide gras.

6. Formulation selon la revendication 5, caractérisée en ce que l'acide gras hydroxylé est la trihydroxystéarine.

7. Formulation selon l'une quelconque des revendications 1 à 6, caractérisée en ce que le médicament est le macrolide C-076.

8. Formulation selon l'une quelconque des revendications 1 à 7, caractérisée en ce que le polymère est un alkyl en $C_{1-4}$ éther ou un ester de cellulose.

9. Formulation selon l'une quelconque des revendications 1 à 8, caractérisée en ce que le polymère a une masse moléculaire de moins de 100 000.

10. Procédé pour la préparation d'un gel pour véhiculer des médications pour animaux qui comporte le mélange d'un polymère soluble dans l'eau avec un médicament pour traiter un état affectant un animal et d'un glycol ou de la glycérine, le chauffage éventuel du mélange et l'addition en portions allant en augmentant avec agitation, d'un ester d'acide gras et de glycérine.

**Revendications pour l'Etat Contractant: AT**

1. Procédé de préparation d'une formulation de gel essentiellement anhydre qui peut être administrée aux animaux, comprenant le mélange homogène de 0,5 à 10% en poids d'un médicament ayant un effet thérapeutique ou prophylactique sur un état affectant un animal et de 0,3 à 15% en poids d'un polymère soluble dans l'eau, avec de 55 à 97,2% en poids d'un glycol ou de glycérine, puis l'addition progressive de 2 à 25% en poids d'un ester d'acide gras hydroxylé et de glycérine.

2. Procédé selon la revendication 1, dans lequel le glycol est le polyéthylèneglycol.

3. Procédé selon la revendication 1, dans lequel le glycol est le propylèneglycol.

4. Procédé selon l'une quelconque des revendications 1 à 3, dans lequel l'ester d'acide gras hydroxylé possède de 10 à 22 atomes de carbone dans le résidu acide gras.

5. Procédé selon la revendication 4, dans lequel l'ester possède de 10 à 18 atomes de carbone et au moins un substituant hydroxyle dans le résidu acide gras.

6. Procédé selon la revendication 5, dans lequel l'acide gras hydroxylé est la trihydroxystéarine.

7. Procédé selon l'une quelconque des revendications 1 à 6, dans lequel le médicament est le macrolide C-076.

8. Procédé selon l'une quelconque des revendications 1 à 7, dans lequel le polymère est un alkyl en $C_{1-4}$ éther ou un ester de cellulose.

9. Procédé selon l'une quelconque des revendications 1 à 8, dans lequel le polymère a une masse moléculaire de moins de 100 00.

10. Procédé pour la préparation d'un gel pour véhiculer des médications pour animaux qui comporte le mélange d'un polymère soluble dans l'eau avec un médicament pour traiter un état affectant un animal et d'un glycol ou de la glycérine, le chauffage éventuel du mélange et l'addition en portions allant en augmentant avec agitation, d'un ester d'acide gras de glycérine.